# FASCICULE DE BREVET EUROPEEN

(11) **EP 3 788 137 B1**
(45) Date de publication et mention de la délivrance du brevet: **19.08.2026**
(21) Numéro de dépôt: 19728488.8
(22) Date de dépôt: 02.05.2019
(51) Int. Cl.: C12N 5/00, A61K 8/49, A61K 8/67, A61K 8/73, A61Q 19/08, A61P 17/02

(54) **COMPOSITION COMPRENANT DE L'ACIDE ALPHA-LIPOIQUE OU L'UN DE SES SELS, UN DERIVE DE VITAMINE C ET DE L'ACIDE HYALURONIQUE ET SON UTILISATION**
ZUSAMMENSETZUNG MIT ALPHA-LIPONSÄURE ODER EINEM ALPHA-LIPONSÄURESALZ, EINEM VITAMIN-C-DERIVAT UND HYALURONSÄURE UND DEREN VERWENDUNG
COMPOSITION COMPRISING ALPHA-LIPOIC ACID OR AN ALPHA-LIPOIC ACID SALT, A VITAMIN C DERIVATIVE AND HYALURONIC ACID AND ITS USE

(30) Priorité: 04.05.2018 FR 1853844; 04.05.2018 FR 1853845
(43) Date de publication de la demande: 10.03.2021
(73) Titulaire: NAOS Institute of Life Science, 13290 Aix-en-Provence (FR); Thorel, Jean-Noël, 75014 Paris (FR)
(72) Inventeur: GATTO, Hugues, 06570 Saint Paul (FR); THOREL, Jean-Noël, 75014 Paris (FR)
(74) Mandataire: Ipsilon
(86) Numéro de dépôt international: PCT/FR2019/051016
(87) Numéro de publication internationale: WO 2019/211567

(56) Documents cités:
- EP-A1- 2 033 689
- WO-A1-2016/057513
- WO-A2-2008/109857
- FR-A1- 2 895 680
- FR-A1- 2 926 461
- FR-A1- 2 954 165
- ANON.: "Molar mass distribution", 13 September 2024 (2024-09-13), XP093285075, Retrieved from the Internet <URL:https://en.wikipedia.org/wiki/Molar_mass_distribution>
- COWMAN M K ET AL: "The content and size of hyaluronan in biological fluids and tissues", FRONTIERS IN IMMUNOLOGY, vol. 6, 2 June 2015 (2015-06-02), XP55672307, ISSN: 1664-3224, DOI: 10.3389/fimmu.2015.00261
- RIVAS F ET AL: "Label-free analysis of physiological hyaluronan size distribution with a solid-state nanopore sensor", NATURE COMMUNICATIONS, vol. 9, no. 1, 1037, 12 March 2018 (2018-03-12), XP093285077, ISSN: 2041-1723, DOI: 10.1038/s41467-018-03439-x
- BAND P A ET AL: "Hyaluronan molecular weight distribution is associated with the risk of knee osteoarthritis progression", OSTEOARTHRITIS AND CARTILAGE, vol. 23, no. 1, January 2015 (2015-01-01), pages 70 - 76, XP093285078, ISSN: 1063-4584, DOI: 10.1016/j.joca.2014.09.017
- "Washing Foam", GNPD, MINTEL, 1 April 2015 (2015-04-01), XP002786697
- "Décolleté Tensing and Firming Care for the Neck", GNPD, MINTEL, 1 February 2017 (2017-02-01), XP002786698

## Description

### DOMAINE DE L'INVENTION

La présente invention concerne une composition comprenant de l'acide α-lipoïque ou l'un de ses sels, un dérivé de vitamine C et de l'acide hyaluronique et son utilisation notamment pour maintenir et/ou induire une croissance cellulaire et/ou induire la synthèse d'éléments cellulaires tels que des éléments de la matrice extracellulaire dermique notamment pour combattre le vieillissement cutané et/ou pour induire une régénération de la peau ou encore son utilisation comme facteur de croissance cellulaire.

### ETAT ANTERIEUR DE LA TECHNIQUE

La peau possède son propre cycle de renouvellement. Les cellules cutanées se régénèrent continuellement au niveau de la couche la plus profonde et migrent vers la surface de la peau pour y remplacer les vieilles cellules. Mais, l'augmentation de l'espérance de vie est associée à un vieillissement physiologique et un ralentissement du taux de renouvellement cellulaire, ce qui provoque, notamment, des modifications de l'état de la peau.

Ce ralentissement de la régénération cellulaire s'accompagne de changements d'ordre esthétique. En effet, avec le temps la peau est plus fine, déshydratée, le taux de collagène diminue se traduisant par l'apparition de rides, une diminution de la fermeté de la peau, ou encore l'apparition d'imperfections.

Deux causes principales sous-tendent l'apparition du vieillissement du tissu cutané, une d'origine intrinsèque et une d'origine extrinsèque.

Le vieillissement cutané intrinsèque est déterminé par une « horloge biologique » individuelle et génétiquement programmée. Ce vieillissement résulte également de la dégradation des systèmes de réparation cellulaire conduisant à l'apparition d'un mécanisme de stress oxydatif et donc à la production des radicaux libres ou espèces réactives de l'oxygène (ROS). En parallèle, d'autres processus sont impliqués dans ce phénomène tels que le raccourcissement des télomères, la sénescence des cellules somatiques, la baisse de niveaux des hormones sexuelles ou encore les modifications de la signalisation intercellulaire. Il s'agit du vieillissement naturel ou chronobiologique.

Le vieillissement extrinsèque, en particulier de la peau, est dû à une exposition aux facteurs environnementaux et aux comportements individuels tels que la pollution, les rayonnements ultraviolets et ionisants, le tabagisme, la consommation d'alcool ou encore une mauvaise alimentation, notamment riche en sucres.

En conséquence de l'exposition à ces facteurs externes, le métabolisme des cellules, spécifiquement des fibroblastes et/ou kératinocytes, diminue et la formation des produits de glycosylation avancés (AGEs) est augmentée, conduisant à dénaturer les protéines du derme (collagène, élastine...). Tous ces mécanismes accélèrent le vieillissement cutané intrinsèque.

Dans le domaine de la cosmétique, de nombreuses compositions ont été développées pour lutter contre le vieillissement cutané, de même que pour favoriser la régénération des cellules de la peau, en particulier chez les personnes âgées. Ces différentes applications sont en général regroupées sous l'indication «anti-âge». Dans ces produits « anti-âge », des ingrédients actifs sont intégrés pour stimuler directement ou indirectement la croissance des fibroblastes et/ou kératinocytes, renforcer la jonction dermo-épidermique, ou encore favoriser la production de collagène et d'élastine.

Parmi les solutions de l'art antérieur, de nombreux composés ne sont pas exempts d'effets secondaires délétères. A titre d'exemple, les rétinoïdes sont décrits pour leur capacité à stimuler la croissance des fibroblastes. Cependant, leur utilisation n'est pas recommandée en cosmétique en raison de leur action phototoxique et potentiellement mutagène (« Photomutagenicity of retinyl palmitate by ultraviolet A irradiation in mouse lymphoma cells » Nan Mei et al. Toxicological sciences 88(1), 142-149 (2005)).

En parallèle, des formulations cosmétiques de l'art antérieur comprennent des facteurs de croissance cellulaire pour leur propriétés anti rides et de synthèse de collagène et d'élastine. A titre d'exemple, le document KR 20180028287 décrit une composition comprenant l'EGF (facteur de croissance épidermique ou « Epidermal Grouwth Factor »), le bFGF (facteur de croissance basique des fibroblastes ou « basic Fibrobalst Growth Factor ») ou encore le KGF (facteur de croissance des kératinocytes ou « Keratinocyte Growth Factor »). Toutefois, ces facteurs de croissance cellulaire sont d'origine animale, cellulaire et/ou végétale, c'est-à-dire obtenus à partir d'une matière première biologique.

Ces facteurs de croissance sont également conventionnellement utilisés en culture cellulaire *in vitro.*

Les facteurs de croissance sont des substances nécessaires à la croissance d'un organisme ou micro-organisme. Il s'agit de suppléments ajoutés à un milieu de culture contenant de nombreuses protéines et facteurs de stimulation du métabolisme cellulaire.

Ces facteurs de croissance sont majoritairement des polypeptides dont l'interaction avec la membrane cellulaire induit, notamment, une réponse hypertrophique (augmentation de la taille des cellules) et une réponse hyperplasique (augmentation de la population cellulaire), c'est-à-dire une prolifération clonale des cellules. En outre, ils favorisent l'adhésion et l'étalement des cellules sur un support.

Les facteurs de croissance cellulaire conventionnels utilisés en culture cellulaire sont d'origine animale, cellulaire et/ou végétale, c'est-à-dire obtenus à partir d'une matière première biologique.

D'autres facteurs largement utilisés en culture cellulaire *in vitro* sont la toxine du choléra, le sérum de veau fœtal (SVF), tout extrait de glande pituitaire de bœuf ou encore les complexes ou fractions indéfinis du lait ou du soja.

Cependant, la composition exacte de ces facteurs de croissance n'est pas clairement identifiée. En d'autres termes, l'importante variété de protéines qu'ils comprennent ne permet pas de connaître leur composition exacte et constitue un frein à la qualité des cultures ou des purifications de protéines d'intérêts sécrétées par les cellules en culture, de même qu'à la reproductibilité des conditions de culture cellulaire *in vitro.*

Un facteur de croissance cellulaire d'origine animale conventionnellement utilisé en culture cellulaire *in vitro* est le SVF. Il s'agit d'un sérum préparé à partir de sang prélevé, par ponction intracardiaque, de fœtus extraits de vaches gestantes.

Un autre facteur de croissance cellulaire utilisé en culture cellulaire *in vitro* est le MPC (« Milk Peptide Complex »). Il s'agit de peptides naturels présents dans des fractions ou extrais obtenus à partir de lait d'origine bovine. À titre d'exemple, le document FR2926461A décrit l'addition d'extrait de laminaire à une base nutritive pour culture cellulaire comprenant du MPC, ainsi que de nombreux composants dont l'acide lipoïque (acide thioctique), l'acide ascobique et l'acide hyaluronique.

Cependant, l'utilisation dans les domaines de la cosmétique ou de la culture cellulaire, de ces facteurs de croissance cellulaire de composition variable, indéfinie et d'origine animale ou végétale est de plus en plus décriée, notamment en raison du manque de traçabilité de ces composants, de la variabilité de leur qualité, d'un risque de contamination et du manque de reproductibilité des effets observés.

De plus, la présence d'allergènes ne peut pas être vérifiée puisque les compositions de ces facteurs de croissance sont indéterminées. Il existe donc un risque d'allergies développées en réponse à leur utilisation.

Pour les facteurs de croissance cellulaire d'origine animale, notamment bovine, une autre source de préoccupation publique s'ajoute et consiste en la présence potentielle de contaminations virales ou de maladies à prions.

A la connaissance du Demandeur, aucun facteur de croissance d'origine non animale, dont la composition est entièrement tracée et traçable, pour induire la croissance cellulaire, notamment des cellules cutanées, n'a été mis au point sans présenter les inconvénients mentionnés précédemment.

Il subsiste un besoin de mettre au point des alternatives, aux rétinoïdes et aux facteurs de croissance, d'origine animale, utilisés notamment dans les compositions cosmétiques de l'art antérieur pour lutter contre le vieillissement cutané.

Un autre problème que se propose de résoudre l'invention est celui de mettre au point un facteur de croissance d'origine non animale dont la composition est entièrement tracée et traçable, notamment pour son utilisation dans les milieux de culture cellulaire *in vitro.*

### EXPOSE DE L'INVENTION

Le Demandeur a constaté qu'il est possible d'induire et de maintenir la croissance des cellules cutanées avantageusement humaines ou animales, de préférence des fibroblastes et/ou des kératinocytes et l'activité de synthèse d'éléments cellulaires, avantageusement constitutifs de la matrice extracellulaire dermique, *via* l'utilisation d'une association spécifique de composés.

Le Demandeur a constaté que, de manière surprenante, cette même association est parfaitement sûre et adaptée à l'utilisation topique, en culture cellulaire *in vitro* ou *ex vivo,* pour l'homme ou l'animal.

Par « éléments cellulaire », on désigne les éléments synthétisés par la cellule, notamment les éléments constitutifs de la matrice extracellulaire dermique tels que l'élastine, le collagène ou encore les glycosaminoglycanes.

Par « facteur de croissance », on désigne un ensemble de substances nécessaires à la survie et/ou la croissance (prolifération, différenciation...) cellulaire, et/ou la synthèse d'éléments (protéines, sucres,...) avantageusement constitutifs de la matrice extracellulaire dermique, par la cellule.

Par « milieu de culture », on désigne un support qui permet la culture des organismes eucaryotes ou procaryotes. Ce milieu fournit les composants indispensables pour la multiplication des cellules. Le terme « milieu de culture » correspond à tout milieu de culture conventionnellement utilisé, tel que les milieux de culture minimum, empirique, sélectif, enrichi, différentiel ou encore discriminant. Il s'agit donc de cultiver des cellules sur un milieu synthétique, en conditions stériles et dans un environnement contrôlé.

Par « culture cellulaire », on désigne un ensemble de techniques biologiques utilisées pour permettre la survie et la croissance d'un tissu ou organe extrait d'un organisme (culture cellulaire *ex vivo)* ou de cellules en dehors de leur milieu d'origine (culture cellulaire *in vitro).*

Ainsi et selon un premier aspect, l'invention a pour objet une composition comprenant :
- de l'acide α-lipoïque ou l'un de ses sels ;
- un dérivé de vitamine C choisi parmi l'éthyle acide ascorbique et l'ascorbate de sodium ou leur mélange ; et
- de l'acide hyaluronique, dont le poids moléculaire moyen en poids (Mw) est compris entre 0,5 et 10 kDa.

L'acide α-lipoïque (no CAS : 1077-28-7), également connu sur le nom d'acide lipoïque ou acide thioctique, possède un atome en C6 qui est chiral. Par conséquent, il existe deux énantiomères de cette molécule : l'acide (R)-(+)-lipoïque et l'acide (S)-(-)-lipoïque, dont le mélange forme le racémique acide (R/S)-lipoïque. L'énantiomère (R) est naturellement synthétisé par les animaux.

Selon un mode de réalisation de l'invention, l'acide α-lipoïque est l'acide (R)-(+)-lipoïque, l'acide (S)-(-)-lipoïque ou leur mélange racémique.

L'acide α-lipoïque est une molécule endogène que l'on retrouve dans tous les organismes eucaryotes. Chez les mammifères, il agit comme un cofacteur d'au moins 4 enzymes mitochondriales qui neutralisent le stress oxydatif *via* une diminution de la production excessive de radicaux libres. Ce composé a donc pour rôle de protéger les protéines contre l'oxydation par les radicaux libres.

Selon un mode de réalisation de l'invention, la composition comprend un sel d'acide α-lipoïque sous forme (R)-(+), (S)-(-) ou bien racémique.

A titre d'exemple, les sels d'acide α-lipoïque qui peuvent être mis en œuvre selon l'invention sont le lipoate de sodium, le lipoate de L-lysine, le lipoate de L-arginine, ou le lipoate de N-methylglucamine.

L'acide α-lipoïque, ou l'un de ses sels, est généralement obtenu par synthèse chimique mais il peut également être obtenu par synthèse à partir de champignons ou de bactéries. Il s'agit d'acide α-lipoïque issu de la biotechnologie. En d'autres termes, il s'agit d'un produit d'origine non animale.

Dans un mode de réalisation préféré, l'acide α-lipoïque, ou l'un de ses sels, peut être utilisé dans la composition de l'invention sous forme purifiée ou isolée. En pratique, l'acide α-lipoïque ou l'un de ses sels peut avoir un degré de pureté, de préférence, au moins égal à 60%, 70%, 80%, 90%, 95% voire au moins égale à 99% en poids.

A titre d'exemple, l'acide α-lipoïque qui peut être mis en œuvre selon l'invention est commercialisé par l'entreprise SABINSA CORPORATION sous le nom d'Alpha Lipoic Acid et correspondant à la dénomination INCI Thioctic acid.

A titre d'exemple, un sel d'acide α-lipoïque qui peut être mis en œuvre selon l'invention est la matière première commercialisée par la société SHANGAI BOYLE CHEMICAL CO., LTD sous le nom Sodium thioctate.

L'éthyle acide ascorbique est disponible sous deux formes, à savoir, le 3-O-Éthyl éther d'acide ascorbique (no CAS 86404-04-8), connu également sous la dénomination 3-O-éthyl éther ascorbyle et le 2- O-Éthyl éther d'acide ascorbique (no CAS 112894-37-8) connu également sous la dénomination 2-O-éthyl éther ascorbyle. Ces deux formes constituent des formes stabilisées de vitamine C (également dénommée acide ascorbique). En d'autres termes, il s'agit de dérivés de vitamine C.

Le 3-O-éthyl éther d'acide ascorbique et le 2-O-éthyl éther d'acide ascorbique sont des précurseurs de l'acide ascorbique, lequel joue un rôle général et fondamental dans la cellule. L'utilisation de ces formes d'éthyle acide ascorbique chez l'Homme est donc parfaitement sûre.

L'éthyle acide ascorbique est généralement obtenu par synthèse chimique mais il peut également être obtenu par synthèse à partir de champignons ou de bactéries. Il s'agit d'éthyle acide ascorbique issu de la biotechnologie. En d'autres termes, il s'agit d'un produit d'origine non animale.

Dans un mode de réalisation préféré, l'éthyle acide ascorbique peut être utilisé dans la composition de l'invention sous forme purifiée ou isolée. En pratique, l'éthyle acide ascorbique peut avoir un degré de pureté, de préférence, au moins égal à 60%, 70%, 80%, 90%, 95% voire au moins égal à 99% en poids.

A titre d'exemple, la matière première Activita commercialisée par la société RIED INTERNATIONAL CORP. et correspondant à la désignation INCI 3-O-ethyl Ascorbic Acid peut être mise en œuvre selon l'invention.

Alternativement, la matière première EVC-2 commercialisée par la société SOGO PHARMACEUTICAL CO. et correspondant à la désignation INCI 2-O-Ethyl Ascorbic Acid peut être mise en œuvre au sens de l'invention.

Dans un mode de réalisation alternatif, un mélange de 3-O-éthyl éther d'acide ascorbique et de 2-O-éthyl éther d'acide ascorbique peut être mis en œuvre selon l'invention.

L'ascorbate de sodium correspond au sel de sodium de la vitamine C et est notamment utilisé comme additif alimentaire pour son effet antioxydant, de préservateur de couleur ou encore comme supplément vitaminique. L'utilisation de l'ascorbate de sodium est donc sûre chez l'Homme.

Dans un mode de réalisation préféré, l'ascorbate de sodium peut être utilisé dans la composition de l'invention sous forme purifiée ou isolée. En pratique, l'ascorbate de sodium peut avoir un degré de pureté, de préférence, au moins égal à 60%, 70%, 80%, 90%, 95% voire au moins égal à 99% en poids.

A titre d'exemple, la matière première Sodium ascorbate USP/FCC commercialisée par la société MCKINLEY RESOURCES, INC. et correspondant à la désignation INCI Sodium ascorbate peut être utilisée dans le cadre de la présente invention.

Selon un mode de réalisation préféré, le dérivé de vitamine C est l'éthyle acide ascorbique, avantageusement le 3-O-éthyl éther d'acide ascorbique.

Pour la suite de la description, par « poids moléculaire (Mw) » on désigne le poids moléculaire moyen en poids (Mw ou Molecular weight).

La composition selon l'invention comprend de l'acide hyaluronique, de préférence de poids moléculaire (Mw) compris entre 0,5 et 10 kDa, ou l'un de ses sels (no CAS :9004-61-9).

Il s'agit d'acide hyaluronique issu de la biotechnologie, notamment par fermentation bactérienne. En d'autres termes, il s'agit d'un produit d'origine non animale.

Dans un mode de réalisation particulier, l'acide hyaluronique de poids moléculaire (Mw) compris entre 0,5 et 10 kDa a un contenu en acide uronique compris entre 4% et 51,3% et un contenu en acide hyaluronique compris entre 88% et 100%.

Dans un mode de réalisation particulier, l'acide hyaluronique de poids moléculaire (Mw) compris entre 0,5 et 10 kDa peut être utilisé dans la composition de l'invention sous forme purifiée ou isolée. En pratique, l'acide hyaluronique de poids moléculaire (Mw) compris entre 0,1 et 15 kDa, avantageusement entre 0,5 et 10 kDa, peut avoir un degré de pureté, de préférence, au moins égal à 60%, 70%, 80%, 90%, 95% voire au moins égal à 99% en poids.

L'acide hyaluronique de poids moléculaire (Mw) compris entre 0,5 et 10 kDa est généralement obtenu par hydrolyse enzymatique ou chimique d'acide hyaluronique natif issu des biotechnologies. D'autres sources d'acide hyaluronique et procédés peuvent être envisagés pour obtenir l'acide hyaluronique de poids moléculaire (Mw) compris entre 0,5 et 10 kDa au sens de l'invention.

Selon l'invention, les hyaluronates, par exemple, le hyaluronate de sodium, sont également des formes d'acide hyaluronique qui peuvent être mises en œuvre dans une composition telle que décrite.

A titre d'exemple, les matières premières Mini HA, Hyalo-oligo, OLIGO-HA, ou Oligohyaferre commercialisées respectivement par les sociétés LEHVOSS, IMCD, SAFIC ALCAN et CONTIPRO peuvent être utilisées dans le cadre de la présente invention.

Selon un autre mode de réalisation, l'acide α-lipoïque, ou l'un de ses sels, représente moins de 0,1% en poids de la composition, avantageusement moins de 0,01%, de préférence entre 0,001% et 0,0005%.

Selon un mode de réalisation particulier, le dérivé de vitamine C selon l'invention représente entre 0,001% et 10% en poids de la composition, avantageusement entre 0,01% et 5%, de préférence entre 0,1% et 1%.

Selon un mode de réalisation particulier, l'acide hyaluronique de poids moléculaire (Mw) compris entre 0,5 et 10 kDa représente entre 0,001% et 10% en poids de la composition, avantageusement entre 0,01% et 5%, de préférence entre 0,1% et 1%.

Selon un mode de réalisation particulier, le dérivé de vitamine C est choisi parmi l'éthyle acide ascorbique et l'ascorbate de sodium ou leur mélange.

Il s'agit avantageusement de l'éthyle acide ascorbique, de préférence le 3-O éthyl éther d'acide ascorbique.

Selon un mode de réalisation particulier, la composition selon l'invention est une composition cosmétique.

Dans un mode de réalisation particulier, la composition selon l'invention, avantageusement cosmétique, comprend, en outre, au moins une forme additionnelle d'acide hyaluronique choisie parmi l'acide hyaluronique de poids moléculaire (Mw) compris entre 20 et 50 kDa ou l'un de ses sels et l'acide hyaluronique de poids moléculaire (Mw) compris entre 100 et 300 kDa (no CAS : 9067-32-7).

A titre d'exemple, les matières premières PRIMALHYAL^{™} 50 ou HyaCare 50 commercialisées respectivement par les sociétés SOLIANCE et EVONIK et correspondant à l'acide hyaluronique de poids moléculaire (Mw) compris entre 20 et 50 kDa peuvent être utilisées dans le cadre de la présente invention.

A titre d'exemple, la matière première PRIMALHYAL^{™} 300 commercialisée par la société SOLIANCE et correspondant à l'acide hyaluronique de poids moléculaire (Mw) compris entre 100 et 300 kDa peut être utilisée dans le cadre de la présente invention.

En pratique, au moins une forme additionnelle d'acide hyaluronique telle que décrite précédemment représente entre 0,001% et 10% en poids de la composition, avantageusement entre 0,01% et 5%, de préférence entre 0,1% et 1%.

Dans un mode de réalisation particulier selon l'invention, la composition, avantageusement cosmétique, comprend en outre de la L-Alanyl-L-Glutamine.

La L-Alanyl-L-Glutamine est un dipeptide naturel qui est présent dans tous les organismes vivants.

De préférence, la L-Alanyl-L-Glutamine peut être utilisée dans la composition de l'invention sous forme purifiée ou hautement purifiée. De préférence, la L-Alanyl-L-Glutamine est obtenue par synthèse chimique.

A titre d'exemple, on peut citer la matière première L-Alanyl-L-Glutamine correspondant à la désignation INCI Alanyl glutamine et commercialisée par la société KYOWA HAKKO KOGYO CO., LTD.

Selon un aspect de l'invention, la L-alanyl-L-glutamine représente entre 0,001% et 5% en poids de la composition, avantageusement entre 0,01% et 2%, de préférence entre 0,1% et 1%.

Dans un mode de réalisation particulier selon l'invention, la composition, avantageusement cosmétique, comprend en outre de la carnosine.

La carnosine est un dipeptide composé à partir de la β-alanine et de l'histidine. Chez l'Homme, on trouve de fortes concentrations de ce composé dans les tissus musculaires et dans le cerveau.

De préférence, la carnosine peut être utilisée dans la composition de l'invention sous forme purifiée ou hautement purifiée. De préférence, la carnosine est obtenue par synthèse chimique.

A titre d'exemple, on peut citer la matière cosmétique Kopsine correspondant à la dénomination INCI Carnosine et commercialisée par l'entreprise KUMAR ORGANIC PRODUCTS LTD.

Selon un aspect de l'invention, la carnosine représente entre 0,001% et 1% en poids de la composition, avantageusement entre 0,01% et 1%.

De manière avantageuse, la composition selon l'invention, avantageusement cosmétique, comprend en outre au moins un composé polyhydroxylé additionnel choisi dans le groupe comprenant le rhamnose, le xylitol, le mannitol et les fructooligosaccharides (FOS).

Dans un mode de réalisation particulier de l'invention, la composition, avantageusement cosmétique, comprend un mélange de deux, trois ou quatre composés polyhydroxylés additionnels cités ci-dessus, de préférence un mélange de rhamnose, xylitol et mannitol.

Avantageusement, le rhamnose représente entre 0,01% et 1% en poids de la composition, le xylitol représente entre 0,05% et 2% en poids de la composition, le mannitol représente entre 0,005% et 1% en poids de la composition et les FOS représentent entre 0,001% et 2% en poids de la composition.

Selon un autre aspect, l'invention concerne une composition avantageusement cosmétique comprenant :
- de l'acide α-lipoïque ou l'un de ses sels ;
- un dérivé de vitamine C choisi parmi l'éthyle acide ascorbique, avantageusement le 3-O éthyl éther d'acide ascorbique, et l'ascorbate de sodium ou leur mélange ; et
- de l'acide hyaluronique avantageusement dont le poids moléculaire (Mw) est compris entre 0,1 et 15 kDa, de préférence entre 0,5 et 10 kDa ;
en outre, au moins un composé choisi parmi :
- du glucose, représentant entre 0,1% et 0,6% en poids de la composition, par exemple 0,45% ;
- de la L-hydroxyproline représentant entre 0,001% et 0,1 % en poids de la composition, par exemple 0,003% ;
- un mélange d'adénosine, de guanine et de ribose représentant chacun entre 0,000001% et 0,0001% en poids de la composition, par exemple 0,00001% ;
- une fraction d'acides aminés, dont certains essentiels, représentant moins de 0,5% en poids de la composition, avantageusement moins de 0,35% ;
- une fraction de vitamines hydrosolubles représentant moins de 0,2% en poids de la composition, avantageusement moins de 0,015% ;
- une fraction inorganique, dont des oligo-éléments et des sels métalliques, représentant moins de 5% en poids de la composition, avantageusement moins de 2%.

Selon un mode préféré de réalisation, le pH de la composition est compris entre 5,0 et 8,0, avantageusement entre 5,5 et 7,9, de préférence entre 7,4 et 7,5, par exemple à une valeur de 7,45 et/ou l'osmolarité est comprise entre 280 et 360 mOsmole, avantageusement entre 300 et 350 mOsmole.

Avantageusement, la composition selon l'invention, de préférence cosmétique, contient d'autres composés, ou « principes actifs », que ceux cités précédemment, en l'espèce l'association de l'acide α-lipoïque, du dérivé de la vitamine C et de l'acide hyaluronique de poids moléculaire (Mw) compris entre 0,5 et 10 kDa ou excipients pour obtenir d'autres effets, de préférence cosmétiques ou dermatologiques, souhaités.

Par « principe actif » on désigne une substance ou un composé qui possède des propriétés biologiques et/ou thérapeutiques qui sous-tendent un effet physiologique.

Le principe actif est à différencier du au moins un excipient, présent dans la composition selon l'invention.

Par « excipient » on désigne une autre substance que le principe actif qui confère à la composition des propriétés, notamment, de consistance, galénique et/ou de vectorisation du principe actif.

Dans un mode de réalisation particulier, la composition selon l'invention contient également des principes actifs aptes à améliorer l'hydratation cutanée.

Dans un mode de réalisation particulier, la composition selon l'invention, avantageusement cosmétique, comprend en outre, de la vitamine B3 (niacinamide ou vitamine PP) ou l'un de ses dérivés.

La vitamine PP, ou l'un de ses dérivés, agit en stimulant l'activité de la sérine palmitoyl transférase, une enzyme impliquée dans la synthèse de sphingosine, molécule précurseur des céramides et de ce fait, améliore la fonction barrière de la peau et combat la sècheresse cutanée. A titre d'exemple, la matière première niacinamide est commercialisée par la société QUIMICA MASSO et correspond à la désignation INCI Niacinamide.

Dans un autre mode de réalisation, la composition selon l'invention, avantageusement cosmétique, comprend en outre, un système bioactif associant d'une part, une forme stable en solution aqueuse d'un nucléotide choisi parmi l'ATP (adenosine triphosphate) le Gp4G (diguanosine tetraphosphate) et l'Ap4A (diadénosine tetraphosphate) ; et d'autre part, au moins un peptide biomimétique comprenant au plus six acides aminés, mimant un polypeptide cutané ou une protéine cutanée, ou une biomolécule agoniste ou antagoniste dudit peptide ou de ladite protéine. En pratique, l'association de ces principes actifs permet de catalyser l'activité métabolique des cellules de la peau tout en obtenant un effet dermocosmétique ou thérapeutique grâce à l'utilisation des peptides biomimétiques. Ces derniers peuvent être sélectionnés afin d'obtenir l'effet souhaité, par exemple, un effet d'inhibition des irritations d'origine neurogène, une activité dépigmentante, un effet inhibant toute intolérance ou sensibilisation etc.

En pratique, dans le système bioactif selon l'invention, le nucléotide représente au plus 10% en poids de la composition, de préférence entre 0,001% et 5% ; et le peptide biomimétique représente entre 0,001% à 1% en poids de la composition.

Selon un autre mode de réalisation, la composition selon l'invention, avantageusement cosmétique, peut comprendre en outre, un extrait de la bactérie *Arthrobacter agilis,* notamment un extrait riche en caroténoïdes. Ainsi la matière première correspondant à la désignation INCI Micrococcus lysate commercialisée par la société GREENTECH peut être utilisée dans le cadre de la présente invention. Avantageusement, la composition selon l'invention comprend entre 0,00001% et 0,1% en poids de la composition, de préférence entre 0,0001% et 0,001% d'un tel extrait sec.

Selon un autre mode de réalisation, la composition selon l'invention, avantageusement cosmétique, comprend en outre d'autres composants pouvant contribuer à la protection interne par une action qui peut consister en une protection de l'ADN, une diminution de l'immunosuppression induite par les radiations UV, une action antiradicalaire ou un effet combiné de ces actions.

L'action protectrice d'une préparation selon l'invention contre le stress oxydatif ou à l'encontre de l'effet des radicaux libres peut être encore améliorée si celle-ci comprend en outre un ou plusieurs antioxydants, aisément sélectionnées par l'Homme du métier par exemple dans la liste suivante : le totarol, le magnolol, l'honokiol, les acides aminés et leurs dérivés, des peptides et leurs dérivés (par exemple l'ansérine, l'hypotaurine, la taurine), les caroténoïdes, les carotènes (α-carotène, β-carotène, lycopène) et leurs dérivés, l'acide chlorogénique et ses dérivés, l'acide dihydrolipoïque, l'aurothioglucose, le propylthiouracile et autres thiols (thiorédoxine, glutathion, cystéine, cystine, cystamine et leurs esters glycosyle, N-acétyle, méthyle, éthyle, propyle, amyle, butyle et lauryle, palmitoyle, oléyle, γ-linoléique, cholestéryle et glycéryle) ainsi que des sels de ceux-ci, le thiodipropionate de dilauryle, le thiodipropionate de distéaryle, l'acide thiodipropionique et ses dérivés, les composés sulfoximine (sulfoximine de buthionine, l'homocystéine sulfoximine, les buthionine sulfones, penta-, hexa- et heptathionine sulfoximine), des agents chélatants (tels que les acides α-hydroxygras, l'acide palmitique, l'acide phytique, la lactoferrine), les α-hydroxyacides (tels que l'acide citrique, lactique, ou malique), l'acide humique, l'acide biliaire, la bilirubine, la biliverdine, le tétraméthylène phosphonate pentasodique d'éthylènediamine et ses dérivés, les acides gras insaturés et leurs dérivés, la vitamine A et ses dérivés (palmitate de vitamine A), le benzoate de coniféryle, l'acide rutinique et ses dérivés, l'α-glycosyl rutine, l'acide férulique et ses dérivés, le furfurylideneglucitol, le butylhydroxytoluène, le butylhydroxyanisole, l'acide nordihydroguaiarétique, trihydroxybutyrophenone, la quercétine, l'acide urique et ses dérivés, le mannose et les dérivés de ceux-ci, le sélénium et ses dérivés (sélénométhionine), les stilbènes et leurs dérivés (l'oxyde de stilbène, l'oxyde trans-stilbène).

Dans un mode de réalisation particulier, la composition selon l'invention, avantageusement cosmétique, contient en outre de l'acide glycyrrhétinique, un dérivé ou un sel de cet acide, utilisé comme apaisant (agent anti-inflammatoire) et représentant entre 0,01% et 2% en poids de la composition, de préférence entre 0,1% et 1%.

Selon un autre mode de réalisation de l'invention, la composition, avantageusement cosmétique, comprend au moins un, voire tous les constituants suivants exerçant une activité biologique *in vivo* sur les cellules de la peau, des lèvres, des cheveux et/ou des muqueuses soumises à un rayonnement UV-A et/ou UV-B, respectivement :
- un agent antiradicalaire préservant les structures cellulaires, tel que par exemple la vitamine E et/ou ses dérivés liposolubles ou hydrosolubles, en particulier le tocotriénol et/ou le tocophérol, représentant avantageusement entre 0,001% et 10% en poids de la composition, encore plus avantageusement entre 0,02% et 2%, de préférence 0,04% ;
- un agent limitant l'immunosuppression, tel que par exemple la vitamine PP, représentant avantageusement entre 0,001% et 1% en poids de la composition, de préférence entre 0,01% et 0,3% ;
- un agent protecteur de la protéine p53, tel que par exemple l'épigallocatéchine gallate (EGCG), représentant avantageusement entre 0,001% et 0,1% en poids de la composition, de préférence entre 0,005% et 0,05%.

En outre, la composition selon l'invention peut également comprendre des extraits peptidiques de soja et/ou de blé.

En pratique, les extraits peptidiques proviennent de graines de soja et de blé sont issus d'une hydrolyse enzymatique desdites graines par l'intermédiaire de peptidases qui permet de récupérer des peptides d'une taille moyenne de 700 Daltons. Préférentiellement, l'extrait peptidique de soja est l'extrait identifié sous le numéro CAS 68607-88-5 de même que l'extrait peptidique de blé est l'extrait identifié sous le numéro CAS 70084-87-6. Les extraits de blé et soja peuvent correspondre aux désignations INCI Hydrolyzed wheat protein et Hydrolyzed soy protein, respectivement.

Dans un mode de réalisation particulier, les extraits peptidiques de soja et de blé sont utilisés ensemble, par exemple dans un rapport pondéral respectivement compris entre 80/20 et 20/80, avantageusement compris entre 70/30 et 30/70, de préférence égal à 60/40.

Dans un mode de réalisation avantageux, les extraits peptidiques de soja et/ou de blé sont exempts de tripeptides synthétiques GHK (glycyl-histidyl-lysine ; INCI : Tripeptide-1). En pratique, les extraits peptidiques de soja et/ou blé représentent entre 0,01% et 20% en poids de la composition, avantageusement entre 0,1% et 10%, de préférence entre 0,2% et 0,7%.

La composition selon l'invention est avantageusement formulée pour être cosmétiquement acceptable, c'est-à-dire compatible avec la peau, les muqueuses, les cheveux et le cuir chevelu. De préférence, la composition de l'invention est une composition pour application cutanée ou à usage topique.

Par « composition pour application cutanée » ou « à usage topique », on désigne une composition compatible avec une application sur la peau, les muqueuses, les cheveux et/ou le cuir chevelu, de préférence la peau humaine.

La composition de l'invention peut se présenter sous toutes les formes galéniques appropriées pour une application topique, notamment sous forme de solution aqueuse, hydroalcoolique, organique ou huileuse ; de suspension ou de dispersion dans des solvants ou des corps gras, de type lotion ou sérum ; sous forme de dispersion vésiculaire ; sous forme d'émulsion eau dans huile (E/H), huile dans eau (H/E) ou multiple telle qu'une émulsion eau dans huile dans eau (E/H/E). L'émulsion peut être plus ou moins épaisse et se présente sous forme de crème ou de lait ; la composition de l'invention peut se présenter également sous forme de pommade, de gel, de bâtonnet solide, de produits anhydres pâteux ou solides, de mousse, notamment aérosol, de composition biphasique ou encore de composition pulvérisable.

La forme galénique de la composition et son mode de préparation, et par conséquent les excipients appropriés à la composition de l'invention, peuvent être choisis par l'Homme du métier sur la base de ses connaissances générales en fonction du type de composition recherchée.

Notamment, la composition peut comprendre tout corps gras usuellement utilisé dans le domaine cosmétique. On peut notamment citer les corps gras siliconés tels que les huiles, les gommes et les cires de silicone, ainsi que les corps gras non siliconés tels que les huiles et les cires d'origine végétale, minérale, animale et/ou synthétique. Les huiles peuvent être volatiles ou non volatiles. On peut encore citer les hydrocarbures, les esters et les éthers de synthèse, les alcools gras et les acides gras. La composition peut également comprendre un milieu aqueux, un milieu hydroalcoolique contenant un alcool tel que l'éthanol ou l'isopropanol, ou un milieu organique comprenant des solvants organiques usuels tels que des alcools en C1-6, notamment l'éthanol et l'isopropanol, des glycols tels que le propylène glycol, des cétones.

La composition peut comprendre au moins un émulsifiant classique, choisi parmi les émulsifiants amphotères, anioniques, cationiques ou non ioniques, utilisés seuls ou en mélange.

Elle peut également comprendre les adjuvants habituels dans le domaine considéré, tels que les épaississants ou gélifiants hydrophiles ou lipophiles, les additifs hydrophiles ou lipophiles, les actifs notamment cosmétiques, les conservateurs, les antioxydants, les parfums, les charges, les pigments, les filtres UV, les absorbeurs d'odeur, les colorants, les hydratants (glycérine), des vitamines, des acides gras essentiels, des polymères liposolubles notamment hydrocarbonés, les opacifiants, les stabilisants, les séquestrants, les conditionneurs et les agents propulseurs.

Dans un mode de réalisation particulier de l'invention, la composition comprend en outre de l'acide hyaluronique de poids moléculaire (Mw) supérieur à 1000 kDa (n° CAS : 9067-32-7), ou l'un de ses sels, en qualité de gélifiant.

A titre d'exemple, la matière première HYALURONATE DE SODIUM PUR commercialisée par la société HTL correspondant à l'acide hyaluronique de poids moléculaire (Mw) supérieur à 1000 kDa peut être utilisée dans le cadre de la présente invention.

Bien entendu, l'Homme du métier veillera à choisir ce ou ces éventuels adjuvants ou excipients complémentaires, et/ou leur quantité, de manière telle que les propriétés avantageuses de la composition selon l'invention ne soient pas, ou substantiellement pas, altérées par l'adjonction envisagée.

Il peut être particulièrement avantageux de formuler la composition de l'invention de façon qu'elle soit pulvérisable. Ceci peut être réalisé par exemple par la formulation d'émulsions spécifiques comprenant des combinaisons particulières d'excipients.

Selon un autre aspect, l'invention concerne également l'utilisation de la composition telle que définie précédemment pour lutter contre le vieillissement de la peau et/ou l'apparition des rides et/ou pour favoriser la régénération de la peau.

Dans ce cadre, la composition selon l'invention assure le maintien de la survie cellulaire et/ou l'induction d'une croissance des cellules cutanées humaines ou animales et/ou d'une activité de synthèse d'éléments cellulaires, avantageusement constitutifs de la matrice extracellulaire dermique notamment le collagène et/ou l'élastine, par des cellules cutanées humaines ou animales, avantageusement des fibroblastes et/ou kératinocytes, de préférence des fibroblastes.

Par «régénération de la peau », on désigne le renouvellement des cellules cutanées, avantageusement des fibroblastes et/ou kératinocytes ainsi que le renouvellement de la matrice extracellulaire produite par lesdites cellules. En d'autres termes, il s'agit de la reconstitution des quantités et de l'activité de cellules cutanées perdues en raison du vieillissement physiologique et du ralentissement du taux de renouvellement cellulaire, et de la stimulation des synthèses de composés cellulaires, notamment fibroblastiques, des composants de la matrice extracellulaire (collagènes, élastine, glycosaminoglycanes ...)

Ainsi et selon un autre aspect, l'invention concerne un procédé de traitement cosmétique des peaux matures consistant à appliquer sur la peau une composition telle que définie ci-dessus.

Avantageusement et en relation avec ces différentes applications, la composition de l'invention est appliquée ou administrée par voie topique.

Selon un autre aspect, l'invention concerne une composition comprenant :
- de l'acide α-lipoïque ou l'un de ses sels ;
- un dérivé de vitamine C choisi parmi l'éthyle acide ascorbique et l'ascorbate de sodium ou leur mélange ; et
- de l'acide hyaluronique,
pour utilisation comme médicament facilitant la cicatrisation de la peau et/ou la guérison des plaies.

Selon un mode de réalisation particulier, la composition selon l'invention pour utilisation comme médicament facilitant la cicatrisation de la peau et/ou la guérison des plaies comprend de l'acide hyaluronique dont le poids moléculaire moyen en poids (Mw) est compris entre 0,5 et 10 kDa.

La composition selon l'invention présente un intérêt majeur dans la promotion de la cicatrisation des plaies et dans la réparation des peaux agressées ou abimées.

La partie d'intérêt à traiter appartient à l'épiderme du sujet et est notamment une zone dudit épiderme présentant un défaut d'aspect en raison d'un défaut naturel, ou d'une lésion tissulaire superficielle non réparée ou réparée, par exemple une zone cicatricielle. Cette partie superficielle d'intérêt peut résulter de toute plaie, balafre, coupure, écorchure, égratignure, entaille, éraflure, morsure, mutilation, piqûre, brûlure, contusion, escarre, ecchymose, en cours de réparation, mais laissant subsister un défaut superficiel, morphologique, structurel, fonctionnel, ou d'aspect. Ainsi, l'invention concerne également l'utilisation de la composition selon l'invention pour induire ou accélérer un processus de cicatrisation d'une plaie topique, avantageusement dermique.

Par « pansement », on désigne tout type de pansements connus et d'une façon préférée les pansements interfaces. De tels pansements sont commercialisés par exemple par SOLVAY PHARMA sous les dénominations commerciales Tulle Gras^{®}.

Ces pansements interfaces se présentent généralement sous forme d'une trame ou d'un filet enduit d'une masse, habituellement une masse élastomérique. Ils peuvent également être constitués d'une masse sans trame ou filet, ayant la forme d'une plaque présentant ou non des trous traversants, en fonction du type de plaie sur lequel le pansement est appliqué (on utilisera préférentiellement une plaque présentant des trous traversants sur une plaie exsudative lorsque la masse n'a qu'un faible ou aucun pouvoir absorbant, les trous permettant ainsi l'évacuation des exsudats de la plaie).

La présente invention trouve également application pour la réalisation de pansements à base d'hydrogels ou d'hydrocolloïdes dans lesquels la composition selon l'invention est incorporée. Des pansements à base d'hydrocolloïdes connus sont par exemple commercialisés par CONVATEC sous les dénominations Duoderm^{®}.

La présente invention trouve également application pour la réalisation de pansements interfaces complexés à une couche absorbante telle qu'une mousse ou une compresse, ou d'une masse hydrocolloïde complexée à une mousse absorbante. De tels pansements sont connus et commercialisés par exemple par les Laboratoires URGO sous les dénominations commerciales UrgotulDuo^{®}.

Selon un autre aspect, l'invention concerne une composition comprenant :
- de l'acide α-lipoïque ou l'un de ses sels ;
- un dérivé de vitamine C choisi parmi l'éthyle acide ascorbique et l'ascorbate de sodium ou leur mélange ; et
- de l'acide hyaluronique,
pour utilisation comme facteur de croissance cellulaire d'origine non animale.

Selon un mode de réalisation particulier, la composition selon l'invention pour utilisation comme facteur de croissance cellulaire d'origine non animale comprend de l'acide hyaluronique dont le poids moléculaire moyen en poids (Mw) est compris entre 0,5 et 10 kDa.

Selon un mode de réalisation particulier, le dérivé de vitamine C compris dans la composition selon l'invention, pour utilisation comme facteur de croissance cellulaire d'origine non animale, est l'éthyle acide ascorbique, avantageusement le 3-O-éthyl éther d'acide ascorbique.

Selon encore un autre aspect, l'invention a pour objet un milieu de culture *in vitro* de cellules cutanées, avantageusement des fibroblastes et/ou des kératinocytes, de préférence des fibroblastes humains ou animaux, comprenant un facteur de croissance cellulaire d'origine non animale comprenant :
- de l'acide α-lipoïque ou l'un de ses sels ;
- un dérivé de vitamine C choisi parmi l'éthyle acide ascorbique, l'ascorbate de sodium ou leur mélange, avantageusement le 3-O éthyl éther d'acide ascorbique ; et
- de l'acide hyaluronique, dont le poids moléculaire moyen en poids (Mw) est compris entre 0,5 et 10 kDa.

Avantageusement, le milieu de culture *in vitro* selon l'invention est déplété en facteur de croissance d'origine animale. En d'autres termes, le seul facteur de croissance cellulaire présent dans le milieu de culture correspond au facteur de croissance cellulaire d'origine non animale selon l'invention.

Avantageusement, l'acide α-lipoïque ou l'un de ses sels représente de moins de 0,1%, avantageusement moins de 0,01% en poids du milieu de culture cellulaire, de préférence entre 0,001% et 0,0005%.

Avantageusement, le dérivé de vitamine C choisi parmi l'éthyle acide ascorbique et l'ascorbate de sodium ou leur mélange, avantageusement l'éthyle acide ascorbique, de préférence le 3-O éthyl éther d'acide ascorbique, représente entre 0,001% et 10% en poids du milieu de culture cellulaire, avantageusement entre 0,01% et 5%, de préférence entre 0,1% et 1%.

Avantageusement, l'acide hyaluronique, dont le poids moléculaire (Mw) est compris entre 0,5 et 10 kDa, représente entre 0,001% et 10% en poids du milieu de culture cellulaire, avantageusement entre 0,01% et 5%, de préférence entre 0,1% et 1%.

Selon un mode de réalisation particulier, le milieu de culture *in vitro* de cellules selon l'invention permet de cultiver des cellules cutanées humaines ou animales, avantageusement des fibroblastes et/ou kératinocytes, de préférence des fibroblastes.

Selon un mode de réalisation particulier, le milieu de culture *in vitro* de cellules cutanées selon l'invention est utilisé pour le maintien de la survie et/ou la croissance cellulaire et/ou la synthèse d'éléments cellulaires, avantageusement constitutifs de la matrice extracellulaire dermique.

Avantageusement, l'invention concerne un milieu de culture *in vitro* de cellules cutanées, avantageusement des fibroblastes et/ou des kératinocytes, de préférence des fibroblastes humains ou animaux, comprenant un facteur de croissance cellulaire d'origine non animale comprenant :
- de l'acide α-lipoïque ou l'un de ses sels;
- un dérivé de vitamine C choisi parmi l'éthyle acide ascorbique, avantageusement le 3-O éthyl éther d'acide ascorbique, et l'ascorbate de sodium ou leur mélange ; et
- de l'acide hyaluronique dont le poids moléculaire (Mw) est compris entre 0,5 et 10 kDa ; en outre, au moins un composé choisi parmi :
- du glucose, représentant entre 0,1% et 0,6% en poids du milieu de culture cellulaire ;
- de la L-hydroxyproline représentant entre 0,001% et 0,1 % en poids du milieu de culture cellulaire ;
- un mélange d'adénosine, de guanine, de ribose représentant chacun entre 0,000001% à 0,0001% en poids du milieu de culture cellulaire ;
- une fraction d'acides aminés, dont certains essentiels, représentant moins de 0,5% en poids du milieu de culture cellulaire, avantageusement moins de 0,35% ;
- une fraction de vitamines hydrosolubles représentant moins de 0,2% en poids du milieu de culture cellulaire, avantageusement moins de 0,015% ;
- une fraction inorganique, dont des oligo-éléments et des sels métalliques, représentant moins de 5% en poids du milieu de culture cellulaire, avantageusement moins de 2%.

Dans un mode de réalisation particulier, le milieu de culture selon l'invention contient en outre tout élément nécessaire à la survie et à la prolifération des cellules.

Par milieu de culture cellulaire au sens de l'invention, on désigne un milieu de culture conventionnel supplémenté en facteur de croissance cellulaire d'origine non animale tel que défini précédemment.

A titre d'exemple, le milieu de culture cellulaire selon l'invention comprend :
- une concentration en glucose d'au moins 0,45% en poids du milieu de culture cellulaire, et par exemple comprise entre 0,1% et 0,6% ;
- une concentration en L-hydroxyproline d'au moins 0,003% en poids du milieu de culture cellulaire, et par exemple comprise entre 0,001% et 0,01% ;
- une concentration en acide ascorbique d'au moins de 0,0001% en poids du milieu de culture cellulaire, et par exemple comprise entre 0,00001% et 0,001% ; et
- une concentration en chacun des composés suivants, à savoir adénosine, guanine, et ribose d'au moins 0,00001% en poids du milieu de culture cellulaire, et par exemple comprise entre 0,000001 % et 0,0001%.

Selon un mode préféré de réalisation, le pH du milieu de culture cellulaire est compris entre 5,0 et 8,0, avantageusement entre 5,5 et 7,9, encore plus avantageusement entre 7,4 et 7,5, par exemple à une valeur de 7,45 et/ou l'osmolarité est comprise entre 280 et 360 mOsmole, avantageusement entre 300 et 350 mOsmole.

Les milieux de culture cellulaire décrits précédemment, supplémentés par le facteur de croissance cellulaire selon l'invention, en l'espèce, l'association de l'acide α-lipoïque, d'un dérivé de la vitamine C et d'acide hyaluronique, sont adaptés à l'utilisation en qualité de milieux de culture, de survie et de croissance de plusieurs lignées cellulaires, avantageusement des cellules cutanées.

A titre d'exemple et de façon non limitative, on peut citer les fibroblastes humains normaux, les lignées fibroblastiques telle que la lignée 3T3, les fibroblastes de cornée de lapin (SIRC), les lignées de fibroblastes pulmonaires humains (WI 38, L 132, Hel 299), la lignée de kératinocytes humains HaCaT, la lignée L 929 issue de tissu conjonctif de souris, la lignée de cellules épithéliales de hamster chinois (CHO-K1), la lignée de mélanome de souris B16F1, la lignée de macrophages murins (Raw 264.7), la lignée humaine d'adénocarcinome utérin HeLa, etc...

Selon un mode de réalisation, le milieu de culture cellulaire selon l'invention, c'est-à-dire supplémenté en facteur de croissance d'origine non animale comprenant l'association de l'acide α-lipoïque, d'un dérivé de la vitamine C et d'acide hyaluronique correspond à la formulation suivante :

| **INGREDIENTS** | **POURCENTAGES (%)** |
|---|---|
| **Composés selon l'invention** | |
| 3-O-ethyl ascorbic acid (dérivé de vitamine C) | 0,2 |
| Hyaluronic acid (acide hyaluronique de poids moléculaire (Mw) compris entre 0,5 et 10 kDa) | 0,2 |
| Thioctic acid (acide α-lipoïque) | 0,00104 |

| **Autres composés** | |
|---|---|
| Sodium Chloride | 0,6885 |
| Alanyl glutamine | 0,20466 |
| Glucose | 0,45 |
| Arginine HCl | 0,04214 |
| Sodium acetate | 0,03 |
| Disodium phosphate | 0,0284 |
| Leucine | 0,01312 |
| Serine | 0,01366 |
| Magnesium chloride | 0,012 |
| Potassium chloride | 0,0112 |
| Valine | 0,00703 |
| Sodium pyruvate | 0,0055 |
| Lysine HCl | 0,007 |
| Histidine HCl | 0,005 |
| Cysteine HCl | 0,0042 |
| Adenine | 0,0019 |
| Threonine | 0,0024 |
| Inositol | 0,002 |
| Glutamic acid | 0,004 |
| Asparagine | 0,00292 |
| Methionine | 0,002 |
| Tyrosine | 0,00117 |
| Phenylalanine | 0,001 |
| Tryptophan | 0,00093 |
| Alanine | 0,00181 |
| Glycine | 0,002 |
| Isoleucine | 0,0006 |
| Aspartic acid | 0,00173 |
| Sodium sulfate | 0,00034 |
| Ferrous sulfate | 0,000139 |
| Folic acid | 0,00018 |
| Thymine | 0,00002 |
| Cyanocobalamin | 0,000041 |
| Calcium pantothenate | 0,00013 |
| Thiamine HCl | 0,00013 |
| Zinc sulfate | 0,0000144 |
| Pyridoxine HCl | 0,000106 |
| Niacinamide | 0,000104 |
| Riboflavin | 0,000014 |
| Biotin | 0,000002 |
| Copper sulfate | 0,0000004 |
| Manganese chloride | 0,0000002 |
| Proline | 0,00461 |
| Hydroxyproline | 0,003 |
| Ascorbic acid | 0,0001 |
| Ascorbate de sodium | 0,0002 |
| Adenosine | 0,00001 |
| Guanine | 0,00001 |
| Ribose | 0,00002 |
| Sodium hyaluronate (sel d'acide hyaluronique de poids moléculaire (Mw) supérieur à 1000 kDa ; agent gélifiant) | 0,07 |
| Carnosine | 0,1 |
| Xylitol | 0,01 |
| Mannitol | 0,01 |
| Rhamnose | 0,005 |
| Fructooligosaccharides | 0,01 |

L'invention concerne également une composition, avantageusement cosmétique, comprenant le milieu de culture *in vitro* selon l'invention.

La composition selon l'invention est pour utilisation pour lutter contre le vieillissement cutané et/ou induire une régénération cellulaire et/ou une cicatrisation des plaies cutanées.

La manière dont l'invention peut être réalisée et les avantages qui en découlent ressortiront mieux des exemples de réalisation qui suivent, donnés à titre indicatif et non limitatif, à l'appui des figures annexées.

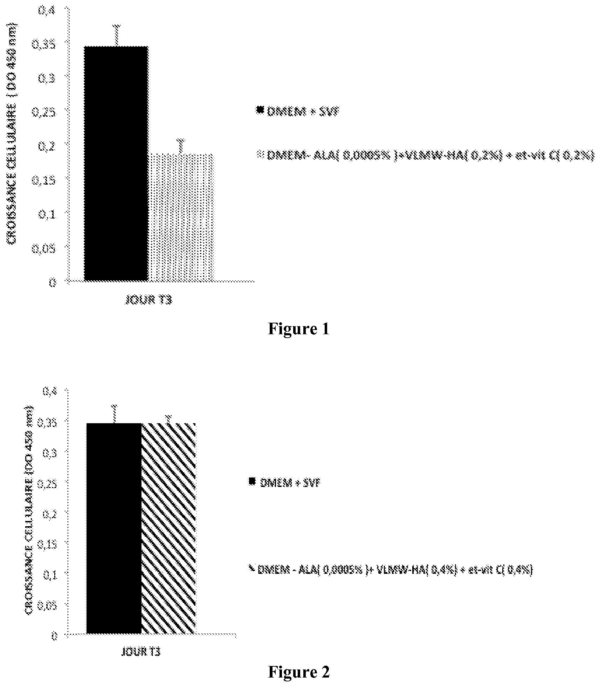

La figure 1 montre l'effet de la combinaison de l'acide α-lipoïque, de l'acide hyaluronique de poids moléculaire (Mw) compris entre 0,5 et 10 kDa, et du 3-O-éthyl éther d'acide ascorbique, respectivement, à des concentrations de 0,0005%, 0,2% et 0,2% en poids de la composition, sur la croissance des fibroblastes humains normaux dans un milieu standard.

DMEM = Dulbecco's Modified Eagle's medium ; SVF = Sérum de veau fœtal ; ALA = acide α-lipoïque ; VLMW-HA = acide hyaluronique de poids moléculaire (Mw) compris entre 0,5 et 10 kDa ; et-vit C= 3-O-éthyl éther d'acide ascorbique.

La figure 2 montre l'effet de la combinaison de l'acide α-lipoïque, de l'acide hyaluronique de poids moléculaire (Mw) compris entre 0,5 et 10 kDa et du 3-O-éthyl éther d'acide ascorbique, respectivement, à des concentrations de 0,0005%, 0,4% et 0,4% en poids de la composition, sur la croissance des fibroblastes humains normaux dans un milieu de culture standard.

DMEM = Dulbecco's Modified Eagle's medium ; SVF = Sérum de veau fœtal ; ALA = acide α-lipoïque ; VLMW-HA = acide hyaluronique de poids moléculaire (Mw) compris entre 0,5 et 10 kDa ; et-vit C= 3-O-éthyl éther d'acide ascorbique.

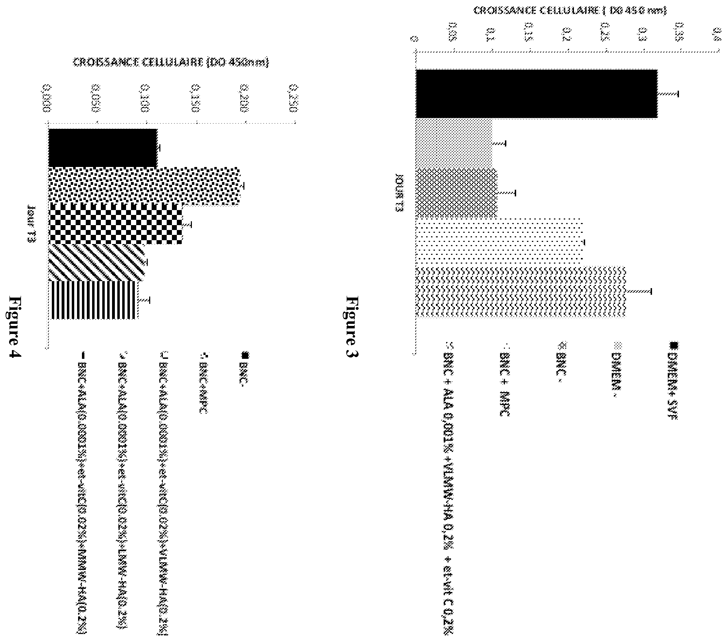

La figure 3 montre l'effet du mélange d'acide α-lipoïque, du 3-O-éthyl éther d'acide ascorbique et d'acide hyaluronique de poids moléculaire (Mw) compris entre 0,5 et 10 kDa, respectivement, à des concentrations de 0,001%, 0,2% et 0,2% en poids du milieu de culture sur la croissance des fibroblastes humains normaux en milieu de culture dit Base Nutritive Complexe.

DMEM- = milieu DMEM déplété ; DMEM + SVF = milieu DMEM contenant du sérum de veau fœtal ; BNC- = milieu BNC déplété ; BNC + MPC = milieu BNC contenant du « Milk Peptide Complex » ; ALA = acide lipoïque ; VLMW-HA= acide hyaluronique de poids moléculaire (Mw) compris entre 0,5 et 10 kDa ; et-vit C = 3-O-éthyl éther d'acide ascorbique.

La figure 4 montre la comparaison de l'effet du mélange d'acide α-lipoïque, du 3-O-éthyl éther d'acide ascorbique et d'acide hyaluronique de différents poids moléculaire (Mw), à savoir compris entre 0,5 et 10 kDa ou compris entre 20 et 50 kDa ou encore compris entre 100 et 300 kDa, respectivement, à des concentrations de 0,001%, 0,2% et 0,2% en poids du milieu de culture sur la croissance des fibroblastes humains normaux en milieu de culture dit Base Nutritive Complexe.

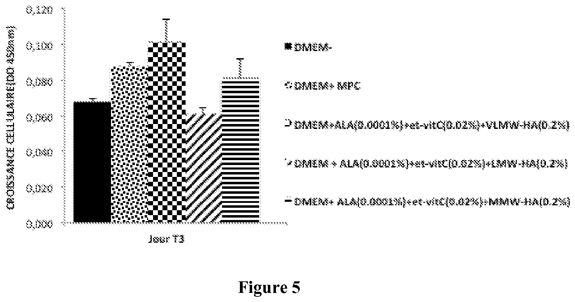

La figure 5 montre la comparaison de l'effet du mélange d'acide α-lipoïque (0,001% en poids du milieu de culture), du 3-O-éthyl éther d'acide ascorbique (0,2% en poids du milieu de culture) et d'acide hyaluronique (0,2% en poids du milieu de culture) de différents poids moléculaire (Mw), à savoir compris entre 0,5 et 10 kDa ou compris entre 20 et 50 kDa ou encore compris entre 100 et 300 kDa, sur la croissance des fibroblastes humains normaux en milieu de culture standard.

DMEM- = milieu DMEM déplété ; DMEM + MPC = milieu DMEM contenant du « Milk Peptide Complex »; BNC- = milieu BNC déplété ; BNC + MPC = milieu BNC contenant du « Milk Peptide Complex » ; ALA = acide lipoïque ; et-vit C = 3-O-éthyl éther d'acide ascorbique ; VLMW-HA= acide hyaluronique de poids moléculaire (Mw) compris entre 0,5 et 10 kDa ; LMW-HA = acide hyaluronique de poids moléculaire (Mw) compris entre 20 et 50 kDa ; MMW-HA= acide hyaluronique de poids moléculaire (Mw) compris entre 100 et 300 kDa.

### EXEMPLES DE REALISATION DE L'INVENTION

### I - Composition cosmétique au sens de l'invention

Les pourcentages indiqués sont donnés en poids de produit par rapport au poids total de la composition.

| **INGREDIENTS** | **POURCENTAGES (%)** |
|---|---|
| **Composés selon l'invention** | |
| 3-O-Ethyl Ascorbic Acid (dérivé de vitamine C) | 0,2 |
| Hyaluronic acid (acide hyaluronique de poids moléculaire (Mw) compris entre 0,5 et 10 kDa) | 0,2 |
| Thioctic acid (acide α-lipoïque) | 0,001 |

| **Autres composés** | |
|---|---|
| Aqua/water/eau | 84,16044 |
| Glycerin | 5 |
| Propanediol | 5 |
| Sodium hyaluronate (sel d'acide hyaluronique de poids moléculaire (Mw) supérieur à 1000 kDa ; gélifiant) | 1,28 |
| Xylitol | 0,9 |
| Sodium chloride | 0,6885 |
| Sodium lactate | 0,54 |
| Glucose | 0,45 |
| *Leuconostoc*/*radish* root ferment filtrate | 0,45 |
| Sodium lauroyl glutamate | 0,245 |
| Alanyl glutamine | 0,20466 |
| Disodium phosphate | 0,1884 |
| *Salix nigra* (willow) bark extract | 0,18 |
| Lactic acid | 0,162 |
| Carnosine | 0,1 |
| Decyl glucoside | 0,05 |

### II Evaluation des effets de la composition selon l'invention sur la croissance de fibroblastes humains normaux dans un milieu standard

### II-1 Eléments d'essai

Les données relatives à la composition selon l'invention testée sont regroupées dans le tableau suivant :

| **Abréviation** | **Dénomination** |
|---|---|
| ALA | Acide α-lipoïque |
| VLMW-HA | Acide hyaluronique de poids moléculaire (Mw) compris entre 0,5 et 10 kDa |
| et-vit C | 3-O-Éthyl éther d'acide ascorbique (dérivé de vitamine C) |

### II-2 Principe

Afin d'évaluer les effets de la composition selon l'invention sur la croissance cellulaire, l'étude est réalisée sur des fibroblastes humains normaux (FHN) ensemencés à faible densité, cultivés en milieu standard déplété en facteur de croissance. Le suivi de la croissance cellulaire est évalué par mesure spectrophotométrique par utilisation du *Cell Proliferation Reagent WST1* (Roche Laboratories).

### II-3 Matériel et méthodes

### i) Milieux et réactifs

Tous les réactifs nécessaires à l'expérimentation sont purs à plus de 95% (analytical grade). Ils ont été manipulés et utilisés en conditions stériles. Du tampon phosphate (PBS, Sigma-Aldrich) a également été employé en cours d'étude.

Un milieu de croissance de cellules standard a été utilisé dans tous les tests pour démontrer l'effet technique de la composition selon l'invention :
- Dulbecco's Modified Eagle's Medium ou DMEM (PAN BioTech DUTSCHER).

### ii) Fibroblastes humains normaux

Les fibroblastes humains normaux néonataux ont été sourcés chez ScienCell Research Laboratories.

### iii) Eléments de référence

- Témoin de croissance cellulaire : Milieu DMEM avec Sérum de Veau Fœtal (SVF) (10% volume : volume)
- Sérum de Veau Fœtal : Facteur de croissance cellulaire standard

### iv) Solubilisation des éléments d'essais

Pour chaque condition testée, une solution mère (SM) comprenant du DMEM non supplémenté avec des facteurs de croissance (DMEM-) est préparée, filtrée à 22µM et conservée à 4°C. Les concentrations préparées pour les solutions mères sont les suivantes :
- Acide α-lipoïque: 0,1%
- Acide hyaluronique de poids moléculaire (Mw) compris entre 0,5 et 10 kDa: 1%
- 3-O-Éthyl éther d'acide ascorbique (dérivé de vitamine C) : 1%.

Les concentrations pour les solutions finales à tester sont préparées par dilution à partir des solutions mères (SM), à savoir :
- Acide α-lipoïque 0,0005% : 50ml de SM qsp10ml de DMEM- ou BNC-
- Acide hyaluronique (quel que soit le poids moléculaire moyen en poids) 0,2% : 2 ml de SM qsp10ml de DMEM- ou BNC-
- Acide hyaluronique (quel que soit le poids moléculaire moyen en poids) 0,4% : 4 ml de SM qsp10ml de DMEM- ou BNC-
- 3-O-Éthyl éther d'acide ascorbique 0,2% : 2ml de SM qsp10ml deDMEM-ou BNC-
- 3-O-Éthyl éther d'acide ascorbique 0,4% : 4 ml de SM qsp10ml deDMEM- ou BNC-

### v) Protocole expérimental

Les fibroblastes humains normaux sont mis en culture, à la densité de 4.10⁴ cellules/ml, dans des plaques de 96 puits (200 µl/puits) en milieu de culture standard des fibroblastes, à savoir dans du DMEM enrichi de 10% de SVF. Ils sont incubés à 37°C, 5% CO₂ pendant 24h. Au 2^{ème} jour, le milieu de culture est éliminé, la composition selon l'invention en solution dans le milieu DMEM- (dépourvu de facteurs de croissance) sont ajoutés dans les puits (200µl /puits) et les cellules sont incubées à 37°C, 5% CO₂.

Chaque condition est réalisée en triplicate. Les milieux ne sont pas renouvelés au cours de l'expérimentation.

La densité cellulaire est évaluée avant la mise au contact avec les différentes conditions d'étude (T0).

La croissance cellulaire des fibroblastes est évaluée au premier (T1), deuxième (T2) et troisième jour (T3) de culture en présence de la combinaison des composés selon l'invention par la technique de conversion du WST-1 qui consiste à évaluer l'activité du système mitochondrial succinate-tetrazolium reductase des cellules vivantes.

Le WST-1 est réduit en formazan qui correspond à un précipité coloré. La viabilité cellulaire est déterminée par lecture spectrophotométrique à 450nm. L'intensité de la densité optique (DO) est proportionnelle au nombre de cellules vivantes et en donne par conséquent une mesure.

### II-4 Résultats

Les résultats obtenus après calcul des moyennes DO lues à 450 nm sont représentés par la figure 1.

La figure 1 représente l'effet de la combinaison l'acide α-lipoïque, l'acide hyaluronique de poids moléculaire (Mw) compris entre 0,5 et 10 kDa et l'éthyle acide ascorbique, à des concentrations respectives de 0,0005%, 0,2% et 0,2% en poids de la composition, sur la croissance des FHN. Le mélange des trois composants démontre une forte stimulation de la croissance cellulaire en milieu déplété en facteur de croissance SVF.

La figure 2 montre qu'en présence des mêmes concentrations d'acide lipoïque mais des concentrations d'acide hyaluronique de poids moléculaire (Mw) compris entre 0,5 et 10 kDa et d'éthyle acide ascorbique de 0,4% en poids de la composition pour chaque composé, une croissance cellulaire comparable à celle obtenue en milieu de culture cellulaire supplémenté en SVF est observée.

### III Evaluation de l'effet de la composition selon l'invention sur la migration cellulaire et la prolifération intervenant dans la recolonisation cicatricielle de plaies.

### III-1 Eléments d'essai

Les données relatives aux composés selon l'invention sont regroupées dans le tableau suivant :

| **Abréviation** | **Dénomination** |
|---|---|
| ALA | Acide α-lipoïque |
| VLMW-HA | Acide hyaluronique de poids moléculaire (Mw) compris entre 0,5 et 10 kDa |
| et-vit C | 3-O-Éthyl éther d'acide ascorbique (dérivé de vitamine C) |

### III-2 Matériel et méthodes

Le test de cicatrisation et d'invasion cellulaire ou « *wound healing assay* » consiste à créer une cicatrice ou brèche au niveau d'un tapis cellulaire de fibroblastes humains normaux et à évaluer au bout d'un temps donné le taux de comblement de la brèche afin de suivre les processus de prolifération et de migration cellulaires participant au phénomène de la cicatrisation. L'analyse des images et la quantification de la densité cellulaire dans la zone de recouvrement de la cicatrice sont réalisées avec l'aide d'un logiciel de traitement et d'analyse d'images.

Les résultats sont exprimés en pourcentages de comblement observé dans chaque brèche. Le taux d'induction est calculé à partir de la différence entre le taux de comblement observé dans l'échantillon et le taux de comblement obtenu dans le témoin négatif.

Les dilutions des composés selon l'invention sont réalisées dans le milieu DMEM déplété de facteurs de croissance (DMEM-).

Le DMEM avec 10% SVF (DMEM avec SVF) constitue le contrôle positif de référence. Conditions étudiées (% en poids de la composition) :
- Acide α-lipoïque 0,001% + Acide hyaluronique de poids moléculaire (Mw) compris entre 0,5 et 10 kDa 0,2% + 3-O éthyle éther d'acide ascorbique 0,2%
- Acide α-lipoïque 0,0005% + Acide hyaluronique de poids moléculaire (Mw) compris entre 0,5 et 10 kDa 0,2% + 3-O éthyle éther d'acide ascorbique 0,4%

Les fibroblastes sont transférés, à une concentration de 1.10⁶ cellules/ml, dans une plaque de 24 puits (500 ml/puits) et incubés à 37°C pendant une nuit. A la fin de la période d'incubation, une plaie est réalisée dans chacun des puits à l'aide d'une pointe de cône pour former les cicatrices et les compositions selon l'invention sont ajoutés. Les cultures cellulaires sont ensuite incubées à 37°C pendant 48h et le comblement des cicatrices est suivi au microscope inversé muni d'un appareil photographique numérique. Un logiciel de traitement et d'analyse d'images permet d'exprimer, par pixellisation, la densité cellulaire dans la zone de comblement.

### III-3 Résultats

Les résultats sont résumés dans le tableau suivant :

| | **% de comblement (pixels noirs)** | | | **% de comblement moyen** | **Taux d'induction /DMEM-(%)** | **Facteur de stimulation/ DMEM - avec SVF** |
|---|---|---|---|---|---|---|
| **DMEM -** | 9,59 | 11,09 | 13,34 | 11,34 ±1,89 | - | - |
| **DMEM avec SVF** | 34,24 | 33,22 | 37,53 | 35,00±2,25 | 23,66 | - |
| **ALA 0,001% + VLMW-HA 0,2% + Et-vit C 0,2%** | **39,75** | **42,18** | **37,07** | **39,66±2,55** | **28,32** | **4,66** |
| **ALA 0,0005% + VLMW-HA 0,2% + Et-vit C 0,4%** | **45,11** | **39,06** | **43,49** | **42,55±3,13** | **31,21** | **7,55** |

Les associations des trois composés selon l'invention à des concentrations différentes telles que mentionnées précédemment stimulent fortement l'invasion et la recolonisation des FHN.

Ces résultats suggèrent que la composition selon l'invention est efficace pour promouvoir et accélérer la cicatrisation des plaies.

### IV - Milieu de culture cellulaire in vitro au sens de l'invention

Les pourcentages indiqués sont donnés en poids de produit par rapport au poids total de la composition.

| **INGREDIENTS** | **POURCENTAGES (%)** |
|---|---|
| **Composés selon l'invention** | |
| 3-O-ethyl ascorbic acid (dérivé de vitamine C) | 0,2 |
| Hyaluronic acid (acide hyaluronique de poids moléculaire (Mw) compris entre 0,5 et 10 kDa) | 0,2 |
| Thioctic acid (acide α-lipoïque) | 0,00104 |

| **Autres composés** | |
|---|---|
| Sodium Chloride | 0,6885 |
| Alanyl glutamine | 0,20466 |
| Glucose | 0,45 |
| Arginine HCl | 0,04214 |
| Sodium acetate | 0,03 |
| Disodium phosphate | 0,0284 |
| Leucine | 0,01312 |
| Serine | 0,01366 |
| Magnesium chloride | 0,012 |
| Potassium chloride | 0,0112 |
| Valine | 0,00703 |
| Sodium pyruvate | 0,0055 |
| Lysine HCl | 0,007 |
| Histidine HCl | 0,005 |
| Cysteine HCl | 0,0042 |
| Adenine | 0,0019 |
| Threonine | 0,0024 |
| Inositol | 0,002 |
| Glutamic acid | 0,004 |
| Asparagine | 0,00292 |
| Methionine | 0,002 |
| Tyrosine | 0,00117 |
| Phenylalanine | 0,001 |
| Tryptophan | 0,00093 |
| Alanine | 0,00181 |
| Glycine | 0,002 |
| Isoleucine | 0,0006 |
| Aspartic acid | 0,00173 |
| Sodium sulfate | 0,00034 |
| Ferrous sulfate | 0,000139 |
| Folic acid | 0,00018 |
| Thymine | 0,00002 |
| Cyanocobalamin | 0,000041 |
| Calcium pantothenate | 0,00013 |
| Thiamine HCl | 0,00013 |
| Zinc sulfate | 0,0000144 |
| Pyridoxine HCl | 0,000106 |
| Niacinamide | 0,000104 |
| Riboflavin | 0,000014 |
| Biotin | 0,000002 |
| Copper sulfate | 0,0000004 |
| Manganese chloride | 0,0000002 |
| Proline | 0,00461 |
| Hydroxyproline | 0,003 |
| Ascorbic acid | 0,0001 |
| Ascorbate de sodium | 0,0002 |
| Adenosine | 0,00001 |
| Guanine | 0,00001 |
| Ribose | 0,00002 |
| Sodium hyaluronate (sel d'acide hyaluronique de poids moléculaire (Mw) supérieur à 1000 kDa ; agent gélifiant) | 0,07 |
| Carnosine | 0,1 |
| Xylitol | 0,01 |
| Mannitol | 0,01 |
| Rhamnose | 0,005 |
| Fructooligosaccharides | 0,01 |

### V - Evaluation de l'effet de la composition selon l'invention sur la croissance de fibroblastes humains normaux dans plusieurs milieux de croissance cellulaire

### V-1 Objectif de l'étude

Le but de l'étude est de comparer l'effet de la composition selon l'invention sur la croissance des fibroblastes humains normaux (FHN) dans le milieu culture BNC, correspondant à un milieu de culture selon l'invention, en comparaison avec les milieux de culture BNC ou DMEM éventuellement supplémentés en facteur de croissance d'origine animale.

### V-2 Eléments d'essai

Les éléments d'essai sont identiques à ceux décrit dans le point II-1.

### V-3 Principe de l'étude

Le principe de l'étude est identique à celui détaillé dans le point II-2.

### V-4 Matériel et méthodes

### i) Milieux et réactifs

Tous les réactifs nécessaires à l'expérimentation sont purs à plus de 95% (analytical grade). Ils ont été manipulés et utilisés en conditions stériles. Du tampon phosphate (PBS, Sigma-Aldrich) a également été employé en cours d'étude.

Milieux de croissance de cellules utilisés:
- Milieu standard "Dulbecco's Modified Eagle's medium" ou DMEM (PAN BioTech DUTSCHER)
- Milieu « éléments vitaux essentiels » ou BNC : la composition de ce milieu, est indiqué dans l'exemple IV ci-dessus.

Le milieu DMEM a été employé sans supplémentation de facteurs de croissance (DMEM-) ou supplémenté avec 10% de Sérum de veau fœtal (DMEM + SVF). L'adjonction de la composition selon l'invention est indiquée dans les figures avec les concentrations utilisées.

Le milieu BNC a été employé sans supplémentation de facteurs de croissance (BNC-) ou bien supplémenté avec 10% de MPC (BNC + MPC). L'adjonction de la composition selon l'invention dans le milieu BNC est indiquée dans les figures avec les concentrations utilisées.

### ii) Fibroblastes humains normaux

Les fibroblastes sont identiques à ceux utilisés pour l'exemple II (voir point II-3. ii).

### iii) Solubilisation des éléments d'essais

Le protocole de solubilisation est identique à celui décrit par le point II-3. iv.

### iv) Protocole expérimental

Le protocole expérimental est identique à celui détaillé par le point II-3. v.

### v) Analyse des résultats

L'absorbance est mesurée à 450 nm. Les valeurs de densité optique (DO), reflet de la croissance cellulaire, sont représentées sous forme d'histogramme.

### V-5 Résultats

Les résultats obtenus après calcul des moyennes de DO lues à 450 nm sont représentés par la figure 3 annexée.

La figure 3 montre l'effet de l'association de l'acide α-lipoïque, de l'éthyl-vitamine C et de l'acide hyaluronique de poids moléculaire (Mw) compris entre 0,5 et 10 kDa sur la croissance des FHN en milieu BNC.

L'association des trois composés (ALA, et-vit C et VLMW-HA) permet d'obtenir une stimulation de croissance cellulaire supérieure au milieu BNC supplémenté avec du MPC et analogue à la croissance observée en milieu DMEM supplémenté avec du SVF.

L'association des composés selon l'invention, présents en milieu de croissance cellulaire conventionnel déplété en facteurs de croissance d'origine animale, induit une croissance cellulaire égale ou supérieure à celle qui est observée en présence d'un facteur de croissance d'origine animale (SVF ou MPC).

### VI - Evaluation de l'effet de l'association de l'acide α-lipoïque et d'un dérivé de vitamine C avec de l'acide hyaluronique de poids moléculaire (Mw) différents sur la croissance de fibroblastes humains normaux dans des milieux de croissance cellulaire

### VI-1 Objectif de l'étude

L'objectif de cette étude est de tester l'effet de l'association de l'acide α-lipoïque, et du dérivé de vitamine C et de trois grades de taille différente, ou poids moléculaire (Mw) différents, d'acide hyaluronique. En particulier, les acides hyaluroniques testés sont un acide hyaluronique de très bas poids moléculaire (entre 0,5 et 10 kDa) ; un acide hyaluronique de bas poids moléculaire (entre 20 et 50 KDa) et un acide hyaluronique de moyen poids moléculaire (entre 100 et 3000 KDa), sur la croissance de fibroblastes humains normaux.

### VI-2 Eléments d'essai

Les données relatives aux composés testés sont regroupées dans le tableau suivant :

| **Abréviation** | **Dénomination** | **Concentration utilisée** |
|---|---|---|
| ALA | Acide α-lipoïque | 0,001% |
| VLMW-HA | Acide hyaluronique de poids moléculaire (Mw) compris entre 0,5 et 10 kDa | 0,2% |
| LMW-HA | Acide hyaluronique de poids moléculaire (Mw) compris entre 20 et 50 kDa | 0,2% |
| MMW-HA | Acide hyaluronique de poids moléculaire (Mw) compris entre 100 et 300 kDa | 0,2% |
| Et-vit C | Ethyl vitamine C (3-O éthyl éther d'acide ascorbique) | 0,2% |

### VI-3 Principe de l'étude

Afin de discriminer l'impact de la nature de l'acide hyaluronique dans l'effet de l'association des 3 composés sur la croissance cellulaire, l'étude est réalisée sur des fibroblastes humains normaux ensemencés à faible densité, cultivés en milieu standard déplété en facteur de croissance. Le suivi de la croissance cellulaire est évalué par mesure spectrophotométrique par utilisation du WST1.

### VI-4 Matériel et méthodes

### i) Milieux et réactifs

Les milieux et réactifs sont conformes à ceux décrits par le point II-3i), avec la seule différence que le milieu DMEM- est également déplété en L-Glutamine stabilisée. Cela permet de mettre mieux en évidence l'effet des différents grades d'acides hyaluroniques sur la croissance des cellules, car la L-Glutamine stabilisée stimule légèrement la prolifération des cellules. L'adjonction des composés selon l'invention est indiquée dans les figures avec les concentrations utilisées.

Le milieu BNC a été employé sans supplémentation de facteurs de croissance (BNC-) ou bien supplémenté avec 0,5% de MPC (BNC + MPC). L'adjonction des composés selon l'invention dans le milieu BNC est indiquée dans les figures avec les concentrations utilisées.

### ii) Fibroblastes humains normaux

Les fibroblastes sont identiques à ceux utilisé pour l'exemple II (voir point II-3.vi).

### iii) Solubilisation des éléments d'essais

Le protocole de solubilisation est identique à celui décrit par le point V-4. i.

### iv) Protocole expérimental

Les fibroblastes humains normaux sont mis en culture, à la densité de 8.104 cellules/ml, dans des plaques de 96 puits (200 µl/puits) en milieu de culture standard des fibroblastes, le milieu DMEM enrichi de 10% de sérum de veau fœtal (SVF).

Ils sont incubés à 37°C, 5% CO₂ pendant 24h. Au 2ème jour, le milieu de culture est éliminé, les composés selon l'invention en solution dans le milieu DMEM- (sans L Glutamine stabilisée) ou BNC- sont ajoutés dans les puits (200µl /puits) et les cellules sont incubées à 37°C ,5% CO2. Parallèlement, des contrôles sont réalisés en milieu DMEM+MPC et milieu BNC+MPC.

Afin de mettre en exergue les effets spécifiques des acides hyaluroniques de poids moléculaire différents, l'acide α-lipoïque, et le dérivé de vitamine C sont utilisés à une concentration 10 fois inférieure à la concentration la plus efficace définie dans les études antérieures (voir point **II-**4), à savoir 0,0001% en poids de la composition pour l'acide α-lipoïque et 0,02% en poids de la composition pour l'éthyl vitamine C. Les acides hyaluroniques de 3 poids moléculaire différents sont testés à la concentration la plus efficace définie antérieurement (voir point II-4), à savoir 0,2% en poids de la composition.

Chaque condition est réalisée en triplicate. Les milieux ne sont pas renouvelés au cours de l'expérimentation.

La densité cellulaire est évaluée avant la mise au contact des composés correspondants aux différentes conditions d'étude (= T0).

La croissance cellulaire des fibroblastes est évaluée au troisième jour (T3) de culture en présence des composés à tester par la technique de conversion du WST-1 conformément au protocole décrit dans l'exemple II-3.

### v) Analyse des résultats

L'absorbance est mesurée à 450 nm. Les valeurs de densité optique (DO), reflet de la croissance cellulaire, sont représentées sous forme d'histogramme.

### VI-5 Résultats

Les résultats obtenus après calcul des moyennes de DO lues à 450 nm sont représentés par les figures 4 et 5 annexées.

De façon surprenante, l'association des trois composés selon l'invention, à savoir ALA, et-vit C et VLMW-HA, permet d'obtenir une stimulation de la croissance cellulaire supérieure à celle obtenue avec les associations ALA, et-vit C et LMW-HA ainsi que ALA, et-vit C et MMW-HA. Cette stimulation est observée à la fois dans le milieu BNC (figure 4) ainsi que dans le milieu DMEM déplété en SVF et L-Glutamine stabilisée (figure 5). Dans le cas du DMEM, la croissance obtenue à T3 avec l'association selon l'invention comprenant les VLMW-HA est supérieure à celle observée en présence d'un facteur de croissance selon l'état de la technique (MPC).

## Revendications

1. Composition comprenant :
- de l'acide α-lipoïque ou l'un de ses sels ;
- un dérivé de vitamine C choisi parmi l'éthyle acide ascorbique et l'ascorbate de sodium ou leur mélange ; et
- de l'acide hyaluronique contenant une fraction dont le poids moléculaire moyen en poids (Mw) est compris entre 0,5 et 10 kDa.

2. Composition selon la revendication 1, **caractérisée en ce que** l'acide α-lipoïque ou l'un de ses sels représente moins de 0,1% en poids de la composition, avantageusement moins de 0,01%, de préférence entre 0,001% et 0,0005%.

3. Composition selon l'une des revendications précédentes, **caractérisée en ce que** le dérivé de vitamine C représente entre 0,001% et 10% en poids de la composition, avantageusement entre 0,01% et 5%, de préférence entre 0,1% et 1%.

4. Composition selon l'une des revendications précédentes, **caractérisée en ce que** l'acide hyaluronique représente entre 0,001% et 10% en poids de la composition, avantageusement entre 0,01% et 5%, de préférence entre 0,1% et 1%.

5. Composition selon l'une des revendications précédentes, **caractérisée en ce que** le dérivé de vitamine C est l'éthyle acide ascorbique, avantageusement le 3-O-éthyl éther d'acide ascorbique.

6. Composition selon l'une des revendications 1 à 5, **caractérisée en ce qu'**il s'agit d'une composition cosmétique.

7. Composition selon l'une des revendications 1 à 6, pour utilisation pour lutter contre le vieillissement de la peau.

8. Composition selon l'une des revendications 1 à 6, pour utilisation comme médicament facilitant la cicatrisation de la peau et/ou la guérison des plaies.

9. Composition selon l'une des revendications 1 à 5, pour utilisation comme facteur de croissance cellulaire d'origine non animale.

10. Utilisation d'un milieu de culture *in vitro* de cellules cutanées comprenant la composition telle que définie par l'une des revendications 1 à 5, comme facteur de croissance cellulaire d'origine non animale.

11. Utilisation d'un milieu de culture *in vitro* de cellules cutanées comprenant la composition telle que définie par l'une des revendications 1 à 5, pour le maintien de la survie et/ou la croissance cellulaire et/ou la synthèse d'éléments cellulaires, avantageusement constitutifs de la matrice extracellulaire dermique.

## Patentansprüche

1. Zusammensetzung, die Folgendes umfasst:
- Alphaliponsäure oder einem Alphaliponsäuresalz;
- ein Vitamin-C-Derivat, ausgewählt aus Ethyl-Ascorbinsäure und Natriumascorbat oder ihrer Mischungen; und
- Hyaluronsäure, die eine Fraktion enthält, deren gewichtsmittleres Molekulargewicht (Mw) zwischen 0,5 und 10 kDa liegt.

2. Zusammensetzung nach Anspruch 1, **dadurch gekennzeichnet, dass** die Alphaliponsäure oder eines der Alphaliponsäuresalze weniger als 0,1 Gew.-% der Zusammensetzung ausmacht, vorteilhafterweise weniger als 0,01 Gew.-%, vorzugsweise zwischen 0,001 % und 0,0005 %.

3. Zusammensetzung nach einem der vorstehenden Ansprüche, **dadurch gekennzeichnet, dass** das Vitamin-C-Derivat zwischen 0,001 Gew.-% und 10 Gew.-% der Zusammensetzung ausmacht, vorteilhafterweise zwischen 0,01 % und 5 %, vorzugsweise zwischen 0,1 % und 1 %.

4. Zusammensetzung nach einem der vorstehenden Ansprüche, **dadurch gekennzeichnet, dass** die Hyaluronsäure zwischen 0,001 Gew.-% und 10 Gew.-% der Zusammensetzung ausmacht, vorteilhafterweise zwischen 0,01 % und 5 %, vorzugsweise zwischen 0,1 % und 1 %.

5. Zusammensetzung nach einem der vorstehenden Ansprüche, **dadurch gekennzeichnet, dass** das Vitamin-C-Derivat Ethyl-Ascorbinsäure ist, vorzugsweise der 3-O-Ethylether der Ascorbinsäure.

6. Zusammensetzung nach einem der Ansprüche 1 bis 5, **dadurch gekennzeichnet, dass** es sich um eine kosmetische Zusammensetzung handelt.

7. Zusammensetzung nach einem der Ansprüche 1 bis 6 zur Verwendung bei der Bekämpfung der Hautalterung.

8. Zusammensetzung nach einem der Ansprüche 1 bis 6 zur Verwendung als Arzneimittel zur Förderung der Hautregeneration und/oder der Wundheilung.

9. Zusammensetzung nach einem der Ansprüche 1 bis 5 zur Verwendung als Zellwachstumsfaktor nichttierischen Ursprungs.

10. Verwendung eines In-vitro-Nährmediums für Hautzellen, das die Zusammensetzung enthält, die in einem der Ansprüche 1 bis 5 definiert ist, als Zellwachstumsfaktor nichttierischen Ursprungs.

11. Verwendung eines In-vitro-Nährmediums für Hautzellen, das die Zusammensetzung enthält, die in einem der Ansprüche 1 bis 5 definiert ist, zur Lebenserhaltung und/oder zum Wachstum von Zellen und/oder der Synthese von Zellbestandteilen, vorzugsweise solchen, die Bestandteil der extrazellulären Matrix der Haut sind.

## Claims

1. Composition comprising:
- α-lipoic acid or one of its salts;
- a vitamin C derivative selected from ethyl ascorbic acid and sodium ascorbate, or a mixture thereof; and
- hyaluronic acid containing a fraction having a weight-average molecular weight (Mw) ranging from 0.5 to 10 kDa.

2. Composition according to claim 1, **characterized in that** the α-lipoic acid or one of its salts represents less than 0.1% by weight of the composition, advantageously less than 0.01%, preferably between 0.001% and 0.0005%.

3. Composition according to any one of the preceding claims, **characterized in that** the vitamin C derivative represents between 0.001% and 10% by weight of the composition, advantageously between 0.01% and 5%, preferably between 0.1% and 1%.

4. Composition according to any one of the preceding claims, **characterized in that** the hyaluronic acid represents between 0.001% and 10% by weight of the composition, advantageously between 0.01% and 5%, preferably between 0.1% and 1%.

5. Composition according to any one of the preceding claims, **characterized in that** the vitamin C derivative is ethyl ascorbic acid, advantageously 3-O-ethyl ascorbic acid ether.

6. Composition according to any one of claims 1 to 5, **characterized in that** it is a cosmetic composition.

7. Composition according to any one of claims 1 to 6, for use in combating skin ageing.

8. Composition according to any one of claims 1 to 6, for use as a medicament promoting skin healing and/or wound healing.

9. Composition according to any one of claims 1 to 5, for use as a non-animal-derived cell growth factor.

10. Use of an *in vitro* culture medium for skin cells comprising the composition as defined in any one of claims 1 to 5, as a non-animal-derived cell growth factor.

11. Use of an *in vitro* culture medium for skin cells comprising the composition as defined in any one of claims 1 to 5, for maintaining cell survival and/or cell growth and/or the synthesis of cellular components, advantageously components constituting the dermal extracellular matrix.
